# EUROPEAN PATENT APPLICATION

(11) **EP 1 950 258 A1**
(43) Date of publication of application: **30.07.2008**
(21) Application number: 06766769.1
(22) Date of filing: 15.06.2006
(51) Int. Cl.: C09C 3/08, A61K 8/19, A61K 8/36, A61K 8/81, A61Q 3/02, A61Q 19/00, C08K 5/09, C08K 9/04, C08L 33/00, C09C 1/00, C09D 7/12, C09D 11/00, C09D 201/00

(54) **ORGANIC INORGANIC COMPOSITE POWDER, PROCESS FOR PRODUCING THE SAME AND COMPOSITION CONTAINING THE POWDER**

(30) Priority: 15.11.2005 JP 2005329814; 15.11.2005 JP 2005329815
(71) Applicant: Pola Chemical Industries Inc., Shizuoka-shi, Shizuoka 422-8009 (JP)
(72) Inventor: TAKASUGA, Yutaka, Yokohama-shi, Kanagawa 244-0812 (JP); TAKAHASHI, Eiji, Yokohama-shi, Kanagawa 244-0812 (JP)
(74) Representative: Bannerman, David Gardner
(86) International application number: PCT/JP2006/312035
(87) International publication number: WO 2007/057997

(57) **Abstract**

The present invention provides an organic inorganic composite powder which has a specific particle shape in accordance with to a washing solvent, which contains fine inorganic particles, and which easily disperses in water by: precipitating a metal salt dissolved in a reaction solvent from the reaction solvent through neutralization or reduction in the presence of a polymer or monomer dissolved in the reaction solvent; polymerizing the monomer after precipitation of the metal salt in the case where the monomer forms no composite with the metal salt during precipitation in the presence of the monomer; washing the obtained precipitate with a specific washing solvent; and drying the resultant.

## Description

### Technical Field

The present invention relates to an inorganic powder composite which is useful in fields of coating materials, cosmetics, and the like and improved dispersibility of fine inorganic particles in water, and to a method of producing the same.

In addition, the present invention relates to a metal oxide in a whisker shape, to a composite of the metal oxide and a polymer, a method of producing the composite, and a cosmetic containing the composite.

### Background Art

Most inorganic particles such as pigments are produced in an aqueous system and are originally hydrophilic. However, the inorganic particles formed in an aqueous system reaction begin particle growth as soon as the particles are formed. Thus, the inorganic particles not only cannot be obtained as very fine particles, but also are formed as a dispersed substance in water, which includes easily aggregated particles and which is hardly re-dispersed, due to polarity. Further, the inorganic particles dried once and formed into powder for easy handling thereafter have activated particle surfaces, and the particles are aggregated with one another through very strong cohesive force, to thereby provide more difficulties in re-dispersion in water having a high polarity.

Meanwhile, there are almost infinite applications employing the inorganic particles such as pigments in an aqueous system, and examples of the applications include an aqueous coating material, paint, cosmetics, food, and aqueous ink. Recently, the inorganic particles to be used in the applications are required to be finer and have good dispersibility by improving the method and device to a higher level and to be a more precision.

For example, titanium oxide or zinc oxide often used as a UV cut material for a sunscreen as cosmetics is originally white powder, forms a white cloudy solution when dispersed in water, and provides white appearance when it is applied on a face or a body. Thus, a method of forming ultrafine particles to improve transparency has been studied vigorously. Further, because a finer pigment for an inkjet printer provides a clearer printed image, an inorganic pigment has recently attracted attention for improving weatherability, and formation of the inorganic pigment into fine particles has been studied vigorously, although the inorganic pigment has not been used because it has a large particle size.

However, in above-mentioned examples, very strong pulverizing force is required for formation of inorganic particles into fine particles to disperse in water, and a large amount of a surfactant is required for dispersion and suppressing re-aggregation thereafter (see Patent Document 1, for example). In this way, fineness of particles is inevitably limited, and applications of the particles are limited depending on the kind and amount of the surfactant at present. Thus, inorganic fine particles more easily dispersed in water are required. That is, it is certain that a fine particle metal oxide or hydroxide having excellent water dispersibility is required.

Meanwhile, an optical effect of a metal oxide or hydroxide as a pigment in a cosmetic is known to be affected by not only its particle size, but also its shape (see Patent Document 2, Patent Document 3, Patent Document 4, and Patent Document 5, for example). That is, a technique of controlling a shape of a metal oxide is useful. Of the techniques of controlling a shape of a metal oxide or hydroxide, a technique of controlling a shape into a fine particle whisker shape is not known at all.
Patent Document 1: JP-A-2001-207060
Patent Document 2: JP-A-2005-289932
Patent Document 3: JP-A-07-157312
Patent Document 4: JP-A-2002-146238
Patent Document 5: JP-A-2005-272466

### Disclosure of the Invention

The present invention has been made in view of the above circumstances, and a first object of the present invention is therefore to provide to an inorganic powder composite of which fine inorganic particles are fine and easily dispersed in water, and a method of producing the same.

A second object of the present invention is to provide a metal oxide or hydroxide which is useful for a cosmetic and is in a fine particle whisker shape.

The present invention provides organic inorganic composite powder (also simply referred to as "powder") having excellent dispersibility in an aqueous system. This powder is in a form of a composite of inorganic fine particles and an organic substance. Examples of such a form include forms in which an organic substance is fixed on inorganic fine particles such as: a form in which a polymer is physically attached on inorganic fine particles; and a form in which inorganic fine particles and a polymer are chemically bonded. The fixation may be determined from a concentration of an organic substance in a washing liquid or from a change in concentration thereof through washing of powder with water, a water-soluble organic solvent, or an aqueous solution of the water-soluble organic solvent.

The inorganic fine particles in the powder of the present invention are fine particles having a particle size of 0.001 to 0.5 µm as a minor axis. However, the powder of the present invention hardly condenses in an aqueous medium such as water or an aqueous solution, and a particle size of the powder and a particle size of the powder dispersed in the aqueous medium (also referred to as "effective particle size") are substantially equal. Further, the powder can be dispersed in the aqueous medium to have an effective particle size similar to the particle size of the powder without specially strong stirring.

The powder of the present invention is obtained by: dissolving a metal salt capable of dissolving in a specific reaction solvent and a polymer capable of dissolving in the reaction solvent in the reaction solvent; exchanging the dissolved metal salt into a salt capable of precipitating from the reaction solvent; washing the obtained solid product with a specific washing solvent; and drying the resultant.

Alternatively, the powder of the present invention is obtained by: dissolving a metal salt capable of dissolving in a specific reaction solvent and a monomer capable of dissolving in the reaction solvent in the reaction solvent; exchanging the dissolved metal salt into a salt capable of precipitating from the reaction solvent; polymerizing the dissolved monomer; washing the obtained solid product with a specific washing solvent; and drying the resultant.

In the present invention, as the reaction solvent, a water-soluble organic solvent, or a mixed solvent of a water-soluble organic solvent and water is employed. Exchange of the dissolved metal salt is performed through neutralization of the metal salt or reduction of a metal. For example, the exchange is performed by: reacting an alkali with the metal salt; and exchanging the metal salt into a metal hydroxide or a metal oxide. Examples of the alkali that can be used include: a known inorganic alkali; and an alkaline organic compound such as an amine or a salt of carboxylic acid with a strong alkali.

In the present invention, polymerization of the monomer may be omitted in the case where the exchanged metal salt and the monomer form a composite. In the case where the monomer is polymerized, a known polymerization initiator in accordance with the kind of monomer may be used.

A particle shape of the powder of the present invention is controlled by the kind of the washing solvent. To be specific, the powder whose particle shape is a spherical shape can be obtained by using water as the washing solvent, and the powder whose particle shape is a whisker shape can be obtained by using an aqueous solution of a water-soluble organic solvent such as a water-containing alcohol as the washing solvent.

In the present invention, washing of the product is preferably performed through decantation from a viewpoint of bringing the washing solvent and the product into contact with each other sufficiently. Washing is preferably performed a plurality of times from the similar viewpoint.

In the present invention, drying of the washed product is not particularly limited so long as it is performed under conditions providing substantially no effects on a state of the obtained powder particles. For example, an excessively high drying temperature may cause aggregation of the particles, and thus drying is preferably performed under mild conditions.

The particle size of the powder of the present invention may be measured by a normal method for measuring a particle size of particles of metal oxide.

Meanwhile, as disclosed in I.M. Ross, C.J. Kiely, and P. Smith "Characteristaion of iron doped zinc oxide using TEM", Inst. Phys. Conf. 147:3, 1995, 95-98, it is known that in the case where metal oxide particles are dispersed in an aqueous medium and a dispersion is measured by a UV spectrometry method, when a particle size in the dispersed state is small, an absorption peak thereof shifts (blue shift) to a short wavelength side from an intrinsic absorption of the metal oxide. Thus, regarding use of such measurement, by comparing an effective particle size of the powder of the present invention and an effective particle size of similar powder except that of the present invention, it can be confirmed that the powder of the present invention has excellent dispersibility in an aqueous system. As specific measurement conditions, a dispersion prepared by dispersing the powder in water is used as a measurement sample, and measurement through UV-vis transmission spectroscopy with a transmission light wavelength within a range of 280 to 450 nm is performed, for example.

A particle shape of the powder of the present invention can be observed with a transmission electron microscope. A mixed ratio of an organic substance and an inorganic substance in the powder of the present invention may be determined through simultaneous differential thermal/calorimetric measurement at 25 to 1,000°C, for example. A structure of inorganic fine particles in the powder of the present invention can be determined through crystal structure analysis with a powder X-ray diffractometer, for example. Further, the particle size of inorganic fine particles in the powder of the present invention may be determined through observation with a transmission electron microscope, for example.

The powder of the present invention has excellent dispersibility in an aqueous system, and thus can be applied to various aqueous compositions each containing powder such as a pigment, such as an aqueous composition containing powder such as a pigment.

Hereinafter, the present invention is disclosed as an invention regarding the powder in a spherical shape as a first invention, and as an invention regarding the powder in a whisker shape as a second invention as below. The description of the first invention may be applied to the second invention, and the description of the second invention may be applied to the first invention within a range not inhibiting an effect of each of the first invention and the second invention.

In the following description, the powder of the present invention is also referred to as an "inorganic powder composite" in the first invention, and is also referred to as a "fine particle metal oxide or hydroxide/water-soluble polymer composite" in the second invention. The metal salt is also referred to as a "water-soluble metal salt" in the second invention. The reaction solvent is also referred to as a "water-miscible organic solvent" or a "mixed liquid of a water-miscible organic solvent and water" in the first invention, and is also referred to as an "aqueous carrier" in the second invention. The polymer is also referred to as a "water-soluble polymer" in the second invention, and the monomer is also referred to as a "water-soluble monomer" in the second invention. The washing solvent is also referred to as an "aqueous carrier" in the second invention.

In view of the circumstances described in the section of Background Art, the inventors of the present invention have conducted intensive studies on an inorganic powder composite of which inorganic particles are fine and which is easily dispersed in water, and have found that an inorganic powder composite which comprises inorganic fine particles and a carboxylic acid derivative represented by a general formula (1) and/or a carboxylic acid derivative polymer represented by the general formula (1) has such features. Thus, the inventors of the present invention have completed the first invention. That is, the first invention is described below.

(1) An inorganic powder composite, comprising as components: inorganic fine particles; and one kind or two or more kinds of compounds selected from the group consisting of a carboxylic acid, a carboxylic acid derivative, a carboxylic acid polymer, and a carboxylic acid derivative polymer each represented by a general formula (1).

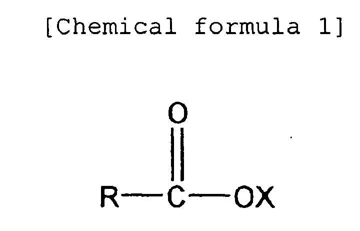

General formula (1)

In the formula: R represents a hydrogen atom, or an alkyl group or alkenyl group which may have one or both of a carboxyl group and a hydroxyl group; and X represents hydrogen, an alkali metal, or polyoxyethylene having 23 or less carbon atoms.

(2) The inorganic powder composite according to the above item (1), in which the carboxylic acid derivative represented by the general formula (1) comprises one kind or two or more kinds of compounds selected from the group consisting of an alkali salt of a mono, di, or tricarboxylic acid having 10 or less carbon atoms, and a polyoxyethylene adduct of a mono, di, or tricarboxylic acid having 10 or less carbon atoms.

(3) The inorganic powder composite according to the above item (1) or (2), in which the carboxylic acid derivative polymer represented by the general formula (1) comprises one kind or two or more kinds of compounds selected from the group consisting of an alkali salt of polyacrylic acid or polymethacrylic acid, and a polyoxyethylene adduct of polyacrylic acid or polymethacrylic acid.

(4) The inorganic powder composite according to any one of the above items (1) to (3), in which: the inorganic fine particles comprises one kind or two or more kinds of compounds selected from the group consisting of a single metal, a metal oxide, and a metal hydroxide; and the metal comprises one kind or two or more kinds of metals selected from the group consisting of zinc, iron, aluminum, magnesium, titanium, barium, manganese, cerium, cobalt, calcium, cadmium, strontium, copper, chromium, zirconium, gold, and silver.

(5) The inorganic powder composite according to any one of the above items (1) to (4), in which: the inorganic fine particles have a particle size of 0.1 µm or less; and the inorganic fine particles are present independently from one another.

(6) The inorganic powder composite according to any one of the above items (1) to (5), in which a percentage of the inorganic fine particles is 60 mass% or more.

(7) An external preparation for skin, comprising the inorganic powder composite according to any one of the above items (1) to (6).

(8) An aqueous nail enamel, comprising the inorganic powder composite according to any one of the above items (1) to (6).

(9) An aqueous ink, comprising the inorganic powder composite according to any one of the above items (1) to (6).

(10) An aqueous coating material, comprising the inorganic powder composite according to any one of the above items (1) to (6).

(11) A method of producing the inorganic powder composite according to any one of the above items (1) to (6), comprising: dissolving a metal salt and one kind or two or more kinds of compounds selected from the group consisting of a lower carboxylic acid represented by the general formula (1) and having X representing hydrogen, a polymer of the lower carboxylic acid, a carboxylic acid derivative represented by the general formula (1) and having X representing an atom excluding hydrogen, and a polymer of the carboxylic acid derivative in a water-miscible organic solvent or a mixed liquid of water and a water-miscible organic solvent; and neutralizing the metal salt or reducing a metal of the metal salt.

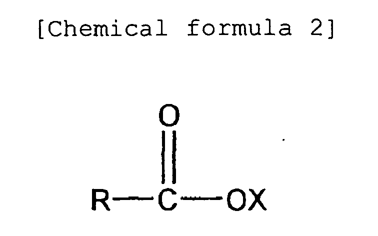

General formula (1)

In the formula (1) : R represents a hydrogen atom, or an alkyl group or alkenyl group which may have one or both of a carboxyl group and a hydroxyl group; and X represents hydrogen, an alkali metal, or polyoxyethylene having 23 or less carbon atoms.

(12) The method according to the above item (11), in which: the compound represented by the general formula (1) comprises one or both of the lower carboxylic acid and a derivative thereof; and the method further comprises polymerizing the lower carboxylic acid or the derivative thereof after neutralization of the metal salt or reduction of the metal.

(13) The method according to the above item (11) or (12), in which the water-miscible organic solvent comprises one kind or two or more kinds of compounds selected from the group consisting of methanol, ethanol, and isopropanol.

(14) The method according to any one of the above items (11) to (13), in which: the lower carboxylic acid represented by the general formula (1) and having X representing hydrogen comprises one kind or two or more kinds of compounds selected from the group consisting of mono, di, and tricarboxylic acids each having 10 or less carbon atoms; and the carboxylic acid derivative represented by the general formula (1) comprises one kind or two or more kinds of polyoxyethylene adducts of lower carboxylic acids each having 10 or less carbon atoms.

(15) The method according to any one of the above items (11) to (14), in which the polymer of the carboxylic acid derivative represented by the general formula (1) comprises one kind or two or more kinds of compounds selected from the group consisting of an alkali salt of polyacrylic acid or polymethacrylic acid, and a polyoxyethylene adduct of polyacrylic acid or polymethacrylic acid.

(16) The method according to any one of the above items (11) to (15), in which the metal salt comprises one kind or two or more kinds of inorganic acid salts selected from the group consisting of zinc, iron, aluminum, magnesium, titanium, barium, manganese, cerium, cobalt, calcium, cadmium, strontium, copper, chromium, zirconium, gold, and silver.

In addition, in view of the circumstances described in the section of Background Art, the inventors of the present invention have conducted intensive studies on a metal oxide which has excellent dispersibility, which is in a form of fine particles, and which can be controlled in shape, and have found that the shape of the metal oxide can be controlled into fine particles and in a whisker shape by producing a metal oxide or hydroxide by the steps of: reacting a metal halide and an alkali in the presence of a water-soluble polymer to form a composite of a metal oxide or hydroxide and the water-soluble polymer; and washing products excluding the composite with a water-containing alcohol; and drying the resultant. Thus, the inventors of the present invention have completed the second invention.

Note that in the second invention, the "fine particles" have a minor axis of 0.5 to 0.001 µm as the metal oxide or hydroxide, and the fine particles do not include the polymer. The composite of the second invention includes: particles in a whisker shape having a polymer as a skeleton and formed by growth of a crystal of metal oxide or hydroxide on a surface of the polymer; and particles in a whisker shape prepared by aggregation of an organic inorganic composite in a form of fiber, having a crystal of metal oxide or hydroxide as a skeleton and a polymer covering surfaces of the metal oxide or hydroxide crystals. In the former particles, the polymer has no substantial effect on the particle size of the composite, and thus the particles of the composite may be regarded as the fine particles.

The composite of the second invention have a major axis of the particles of 0.01 to 50 µm. The whisker shape in the second invention refers to shape in which a ratio of the major axis to minor axis of the particles is 10 or more. An upper limit for the ratio is not particularly limited, but is generally about 100, and preferably about 70.
The second invention is described below.

(17) A method of producing a fine particle metal oxide or hydroxide/polymer composite, comprising the steps of: reacting a metal salt and an alkali in the presence of a water-soluble polymer, or reacting a metal salt and an alkali in the presence of a water-soluble monomer and then polymerizing the water-soluble monomer, to form a composite of a metal oxide or hydroxide and the water-soluble polymer; washing products excluding the composite with a water-containing alcohol; and drying the resultant.

(18) The method according to the above item (17), in which a fine particle metal oxide in the fine particle metal oxide or hydroxide/polymer composite is in a whisker shape.

(19) The method according to the above item (17) or (18), in which a fine particle metal oxide in the fine particle metal oxide or hydroxide/polymer composite comprises zinc oxide.

(20) The method according to any one of the above items (17) to (19), in which the water-soluble polymer comprises polyacrylic acid and/or a salt thereof, polymethacrylic acid and/or a salt thereof, or polyvinyl alcohol.

(21) A fine particle metal oxide or hydroxide/polymer composite comprising a composite of a water-soluble polymer and a fine particle metal oxide or hydroxide, in which the metal oxide or hydroxide is present in a whisker shape.

(22) The fine particle metal oxide or hydroxide/polymer composite according to the above item (21), in which the water-soluble polymer comprises polyacrylic acid and/or a salt thereof, polymethacrylic acid and/or a salt thereof, and polyvinyl alcohol.

(23) The fine particle metal oxide or hydroxide/polymer composite according to the above item (21) or (22), in which a fine particle metal oxide in the fine particle metal oxide or hydroxide /polymer composite comprises zinc oxide.

(24) A fine particle metal oxide or hydroxide, which is a metal oxide or hydroxide in a whisker shape.

(25) The fine particle metal oxide or hydroxide according to the above item (24), in which the fine particle metal oxide or hydroxide forms a composite with a water-soluble polymer and is present on the water-soluble polymer.

(26) The fine particle metal oxide or hydroxide according to the above item (24) or (25), in which the fine particle metal oxide or hydroxide comprises zinc oxide.

(27) An external preparation for skin, comprising the fine particle metal oxide or hydroxide/polymer composite according to any one of the above items (21) to (23).

(28) The external preparation for skin according to the above item (27), in which the external preparation for skin is in a water-containing form.

(29) The external preparation for skin according to the above item (27) or (28), in which the external preparation for skin is a cosmetic.

The first invention can provide an inorganic powder composite of which inorganic particles are fine and easily dispersed in water, and a method of producing the composite.

The second invention can provide a fine particle metal oxide or hydroxide/polymer composite in a whisker shape or a metal oxide or hydroxide in a fine particle and in a whisker shape ,which is useful for a cosmetic.

### Brief Description of the Drawings

[Fig. 1] A diagram showing Composite 1a of Example 1 (photograph substituted for drawing).
[Fig. 2] A diagram showing Composite 1b of Example 11 (photograph substituted for drawing).
[Fig. 3] A diagram showing Composite 2b of Example 13 (photograph substituted for drawing).
[Fig. 4] A diagram showing Composite 3b of Example 15 (photograph substituted for drawing).

### Best Mode for carrying out the Invention

### (1) Inorganic powder composite of first invention

An inorganic powder composite of the first invention is characterized by including: inorganic fine particles; and one kind or two or more kinds of compounds selected from the group consisting of a carboxylic acid, a carboxylic acid derivative, a carboxylic acid polymer, and a carboxylic acid derivative polymer (hereinafter, also referred to as "carboxylic acid and the like" collectively) each represented by a general formula (1).

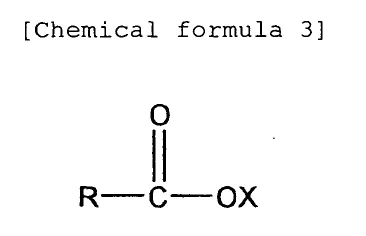

General formula (1)

In the general formula (1), R represents a hydrogen atom, or an alkyl group or alkenyl group which may have one or both of a carboxyl group and a hydroxyl group. In the general formula (1), X represents hydrogen, an alkali metal, or polyoxyethylene having 23 or less carbon atoms. In the present invention, the alkyl group and the alkenyl group may each have a straight chain structure or a branched structure, and an appropriate substituent such as a hydrogen atom may be bonded to a terminal of the polyoxyethylene.

The inorganic powder composite of the first invention formed of the inorganic fine particles and the carboxylic acid and the like each represented by the general formula (1) may have a molecule releasing through formation of a composite of the two components. Examples of the releasing molecule include water, an alcohol, an alkali metal salt, and an alkali metal hydroxide.

The particle size as used herein refers to a maximum diameter of the inorganic fine particles, and is 0.1 µm or less. The range of the particle size varies depending on applications. The particle size of the inorganic fine particles is about 0.1 to 0.01 µm for coloring, and the particle size thereof is about 0.01 to 0.001 µm for emphasizing transparency and requiring functions in a UV absorbing agent, a disinfectant, or the like. Further, the particle size of the inorganic powder composite of the first invention varies depending of applications. The particle size of the inorganic powder composite is preferably 50 to 0.02 µm for coloring, and the particle size thereof is about 5 to 0.002 µm for a UV absorbing agent, a disinfectant, or the like. In the first invention, a spherical shape of the particles of the inorganic powder composite can be observed from appearance of the inorganic powder composite with a scanning electron microscope. The term "spherical shape" includes not only a perfectly spherical shape, but also a substantially spherical shape. In the first invention, all of the inorganic powder composite need not have a spherical shape, and about 2/3 or more of the inorganic powder composite preferably has a spherical shape. Regarding the particle shape of the inorganic powder composite, a ratio of a minimum diameter to a maximum diameter of the inorganic powder composite is preferably 0.6 to 1.0, and more preferably 0.8 to 1.0.

The inorganic fine particles are present independently from one another in the inorganic powder composite of the first invention. The particle size of the fine particles and the state of the particles in the composition can be observed with a transmission electron microscope, and are adjusted by the kind of inorganic substance and the production method described in detail below.

Next, as a composition ratio of the inorganic fine particles to the carboxylic acid and the like each represented by the general formula (1) of the first invention, the inorganic fine particles are desirably 60% or more in mass percentage. The percentage cannot be determined generally because dispersibility in water varies depending on the kind or particle size of the inorganic fine particles. The percentage is preferably 60% to 99%, and more preferably 85% to 99%.

The kind of inorganic substance to be used herein is not particularly limited, but if we venture to say, examples thereof include zinc, iron, aluminum, magnesium, titanium, barium, manganese, cerium, cobalt, calcium, cadmium, strontium, copper, chromium, zirconium, gold, and silver. The inorganic substance is used in a form of a single substance, an oxide, or/and a hydroxide, and is used as one kind or a composite of two or more kinds thereof.

Examples of the carboxylic acid derivative represented by the general formula (1) include: an alkali salt such as potassium, sodium, or lithium salt, or an amine; and a polyoxyethylene adduct; of a monocarboxylic acid, a dicarboxylic acid, or a tricarboxylic acid. Of those, a carboxylic acid (hereinafter, also simply referred to as an "aliphatic acid") having 10 or less carbon atoms has excellent miscibility with water and is particularly desired.

Examples of the alkali salt include sodium acetate, potassium propionate, sodium acrylate, triethylamine methacrylate, sodium caproate, lithium oxalate, potassium malonate, sodium succinate, potassium citrate, and sodium tartrate. Examples of the polyoxyethylene adduct include polyoxyethylene acrylate and polyoxyethylene methacrylate.

Examples of the carboxylic acid derivative polymer represented by the general formula (1) include: sodium polyacrylate, triethanolamine polyacrylate, sodium polymethacrylate, and triethylamine polymethacrylate as alkali salts; and a polyoxyethylene acrylic polymer and a polyoxyethylene methacrylic polymer each having 23 moles or less of oxyethylene chains as polyoxyethylene adducts. A polymerization degree of the polymer is preferably 1, 000 or less.

The inorganic powder composite can be used as a pigment or a UV scattering agent in an aqueous composition containing inorganic powder such as an external preparation for skin such as a cosmetic, ink, or a coating material in the same manner as known inorganic powder.

### (2) Method of producing inorganic powder composite of first invention

A method of producing an inorganic powder composite of the first invention is characterized by including: dissolving a lower aliphatic acid represented by the general formula, derivative thereof, lower aliphatic acid polymer, derivative thereof, or a polymer of a derivative of the lower aliphatic acid, and a metal salt in a water-soluble organic solvent (also referred to as a "water-miscible organic solvent") or a mixed liquid of water and a water-soluble organic solvent; neutralizing the metal salt or reducing a metal of the metal salt; and polymerizing the lower aliphatic acid or the derivative thereof as required.

In general, in a step of hydrolyzing a metal salt in an aqueous system to form a hydroxide or an oxide, it is known that mixing of a small amount or large amount of an organic solvent thereto converts a hydrolyzed product of the metal salt into an oxide. As the water-soluble organic solvent to be used in the production method of the first invention, almost any organic solvent, used for directly converting the hydrolyzed product of the metal salt into an oxide and capable of mixing with water, may be used.

Examples of such organic solvent include: alcohols such as methanol, ethanol, and isopropanol; diols such as ethanediol, propanediol, and butanediol; ketones such as acetone; furans such as tetrahydrofuran; ethylene glycols each having a molecular weight of 200 or less; and ethylene glycol monoethers such as methoxyethanol and ethoxyethanol.

A mixed ratio of the water-soluble organic solvent in the mixed liquid cannot be defined generally because the ratio varies depending on the kinds of lower aliphatic acid, derivative thereof, a polymer of the lower aliphatic acid, derivative thereof, or a polymer of a derivative of the lower aliphatic acid, and metal salt, and on the kinds of water-soluble organic solvent to be used and reaction side products. The ratio of water:water-soluble organic solvent is within a range of about 1:9 to 9:1 in weight ratio. After completion of the reaction, washing with water or the like is preferably performed to remove an excess salt.

As the lower aliphatic acid, derivative thereof, a polymer of the lower aliphatic acid, derivative thereof, or a polymer of a derivative of the lower aliphatic acid to be used in the production method of the first invention, a carboxylic acid and the like each represented by the general formula (1) may be used. As described above, examples of the lower aliphatic acid or the derivative thereof include: a monocarboxylic acid, a dicarboxylic acid, or a tricarboxylic acid; a salt thereof; and a polyoxyethylene adduct of the carboxylic acid. Of those, a carboxylic acid having 10 or less carbon atoms has excellent miscibility with water and a water-soluble organic solvent, and is particularly desired.

Examples of the lower aliphatic acid include acetic acid, propionic acid, acrylic acid, methacrylic acid, capronic acid, oxalic acid, malonic acid, succinic acid, citric acid, and tartaric acid. An example of the derivative thereof is a polyoxyethylene adduct having 23 moles or less of oxyethylene chains such as polyoxyethylene acrylate or polyoxyethylene methacrylate. Examples of the polymer of the lower aliphatic acid include polyacrylic acid and polymethacrylic acid. Examples of the derivative thereof or the polymer of a derivative of the lower aliphatic acid include polyoxyethylene acrylic polymer and polyoxyethylene methacrylic polymer having 23 moles or less of oxyethylene chains of polyoxyethylene adducts.

Neutralization of the metal salt or reduction of the metal may be performed by: adding a water-soluble base such as sodium hydroxide; or using a strong basic salt of a carboxylic acid exhibiting basic such as trisodium citrate for the carboxylic acid and the like each represented by the general formula (1). Drying of the product after washing may be performed in the same manner as that in the second method described below.

The production method of the first invention may include a step of polymerizing the lower aliphatic acid or the derivative thereof as required after neutralization of the metal salt or reduction of the metal. Examples of the case requiring polymerization include a case where one or both of the carboxylic acid and the derivative thereof is used for the compound represented by the general formula (1) to desire to obtain an organic inorganic composite powder which comprises a polymer of the carboxylic acid or a polymer of the carboxylic acid derivative and the metal salt after neutralization of the metal salt or reduction of the metal, that is, a case where a monomer and a metal are formed into a composite at first, and then the monomer is polymerized to thereby obtain a composite of a polymer and the metal. It is preferable to contain such a polymerization step for obtaining composite powder eventually composed: a polymer having physical properties providing difficulties in handling of the polymer alone such as high viscosity and low solubility; and a metal.

(3) Composite of fine particle metal oxide or hydroxide/water-soluble polymer (hereinafter, also simply referred to as "composite") of second invention
A composite of the second invention is produced by: neutralizing a water-soluble metal salt, such as a halide such as a chloride or a nitrate, with a water-soluble base in an aqueous carrier in the presence of a water-soluble polymer; and ion exchanging anion residues of the metal salt and hydroxide ions.

Alternatively, a composite of the second invention is produced by: neutralizing the water-soluble metal salt with a water-soluble base in an aqueous carrier in the presence of a water-soluble monomer to ion-exchang anion residues of the metal salt and hydroxide ions; and polymerizing the water-soluble monomer.

Examples of the metal forming the metal oxide or hydroxide include zinc, iron, aluminum, magnesium, titanium, barium, manganese, cerium, cobalt, calcium, cadmium, strontium, copper, chromium, zirconium, gold, and silver. Of those, a metal belonging to an amphoteric metal is preferred, and examples thereof include zinc and aluminum. In particular, zinc is preferred from an optical effect. One kind of metal salt may be used, or two or more kinds thereof may be used in a form of a composite metal oxide or hydroxide.

The water-soluble polymer is not particularly limited so long as the polymer is capable of "dissolving" in transparent in water, but preferably has a carboxyl group or a group containing a salt thereof. Specific preferred examples thereof include a polymer formed of acrylic acid or methacrylic acid as a monomer component, a copolymer, and/or salts thereof.

The term "dissolving" refers to providing uniform distribution. Preferred examples of the salt of the polymer include alkali metal salts such as a sodium salt and a potassium salt. Preferred examples of other water-soluble polymer include alginic acid and/or a salt thereof, and carboxymethyl cellulose and/or a salt thereof.

Mass of the metal salt and the water-soluble polymer is preferably set such that the metal salt is 60 mass% or more, preferably 60% to 99% or more, and more preferably 85% to 99% when the metal salt is converted into a metal oxide or hydroxide.

The aqueous carrier may contain water, and is preferably used by mixing with an organic solvent which is soluble in water. In general, in a step of hydrolyzing a metal salt in an aqueous system to form a hydroxide or an oxide, it is known that mixing of a small amount or large amount of an organic solvent thereto converts a hydrolyzed product of the metal salt into an oxide. As the water-soluble organic solvent to be used in the production method of the second invention, almost any organic solvent, used for directly converting the hydrolyzed product of the metal salt into an oxide and capable of mixing with water, may be used.

Examples of such organic solvent include: alcohols such as methanol, ethanol, and isopropanol; diols such as ethanediol, propanediol, and butanediol; ketones such as acetone; furans such as tetrahydrofuran; ethylene glycols each having a molecular weight of 200 or less; and ethylene glycol monoethers such as methoxyethanol and ethoxyethanol.

Regarding a mixed ratio of such water-soluble organic solvent, the ratio of water: water-soluble organic solvent is within a range of about 1: 9 to 9:1 in weight ratio. After completion of the reaction, washing with water-containing alcohol or the like is preferably performed to remove an excess salt.

Preferred examples of the base for forming the metal oxide or hydroxide include alkali metal hydroxides such as sodium hydroxide and potassium hydroxide. An addition amount of the base is preferably equal to or slightly more than the amount of the metal salt.

The composite obtained after a while through a reaction of the water-soluble polymer, the water-soluble metal salt, and the base in an aqueous carrier precipitates through centrifugation or the like. The precipitate is washed with an aqueous carrier containing the organic solvent once or several times, so unnecessary reaction products can be removed.

The aqueous carrier used for washing the precipitate contains water and the organic solvent. A percentage of water in the aqueous carrier for washing is preferably 10 to 90 vol%, and more preferably 30 to 70 vol% though depending on the kind of organic solvent to be mixed. In the case where the aqueous carrier for washing is a water-containing alcohol, a water content is preferably 20 to 80 vol%, and more preferably 30 to 70 vol%. The kind of alcohol is not particularly limited so long as it is water-soluble, and one kind of alcohol or two or more kinds thereof may be used.

After such treatment, the precipitate is dried, so the composite of the second invention can be obtained. The drying is performed by air drying under heating at 30 to 100°C for about 1 to 24 hours.

After a while, a composite including the water-soluble polymer and the metal oxide or hydroxide in a whisker shape entangled together is obtained. The composite includes the composed water-soluble polymer, and thus the composite alone has an excellent action of uniformly dispersing in an aqueous carrier. The composite is in a whisker shape, and thus does not provide very white appearance and has high transparency. The composite has an excellent UV-protecting effect, and thus is preferred as a raw material for a UV-protecting cosmetic without providing white appearance.

The composite is calcined at 500 to 1,000°C in an oxidizing atmosphere, so the water-soluble polymer can be burned off, and a metal oxide in a whisker shape may be formed.

### (4) External preparation for skin of second invention

An external preparation for skin of the second invention is characterized by including the composite of the second invention. A preferred content of the composite in a cosmetic of the second invention is preferably 0.1 to 30 mass%, and more preferably 1 to 20 mass% in total. The content varies depending on the form and kind of the external preparation for skin, but the content of the composite within the above ranges provides an excellent UV-protecting effect without unnatural finish with white appearance when the external preparation for skin is used.

As the external preparation for skin of the second invention, a commonly known powder-containing external preparation for skin may be used without particular limitation. Preferred examples of the external preparation for skin of the second invention include: a UV-protecting cosmetic such as sun care milk, sun care powder, or sun block; and a makeup cosmetic such as under makeup, foundation, control color, or pressed powder. Especially preferred examples of the external preparation for skin of the second invention include a summer makeup cosmetic and the like. Regarding a form thereof, the external preparation for skin of the second invention may be applied to any form of: a two-layer dispersion lotion, emulsification, powder, oil, or the like. Particularly preferred form includes a two-layer dispersion lotion and emulsification each containing an aqueous carrier.

The external preparation for skin of the second invention may contain an arbitrary component used in a general external preparation for skin in addition to the composite of the second invention.

Preferred examples of such arbitrary component include: oils and waxes such as macadamia nut oil, avocado oil, corn oil, olive oil, rapeseed oil, sesame oil, castor oil, safflower oil, cottonseed oil, jojoba oil, coconut oil, palm oil, liquid lanoline, hydrogenated coconut oil, hydrogenated oil, Japan wax, hydrogenated castor oil, bees wax, candelilla wax, carnauba wax, ibota wax, lanoline, hydrogenated lanoline, hard lanoline, and jojoba wax; hydrocarbons such as liquid paraffin, squalane, pristane, ozokerite, paraffin, ceresin, vaseline, and microcrystalline wax; higher fatty acids such as oleic acid, isostearic acid, lauric acid, myristic acid, palmitic acid, stearic acid, behenic acid, and undecylenic acid; higher alcohols such as cetyl alcohol, stearyl alcohol, isostearyl alcohol, behenyl alcohol, octyl dodecanol, myristyl alcohol, and cetostearyl alcohol; synthetic ester oils such as cetyl isooctanoate, isopropyl myristate, hexyldecyl isostearate, diisopropyl adipate, di-2-ethylhexyl sebacate, cetyl lactate, diisostearyl malate, ethylene glycol di-2-ethylhexanoate, neopentyl glycol dicaprate, glycerin di-2-heptyl undecanoate, glycerin tri-2-ethylhexanoate, trimethylolpropane tri-2-ethylhexanoate, trimethylolpropane triisostearate, and pentaerythritol tetra-2-ethylhexanoate; a chain polysiloxane such as dimethylpolysiloxane, methylphenylpolysiloxane, or diphenylpolysiloxane; a cyclic polysiloxane such as octamethylcyclotetrasiloxane, decamethylcyclopentasiloxane, or dodecamethylcyclohexane siloxane; oils such as silicone oils including modified polysiloxanes such as amino-modified polysiloxane, polyether-modified polysiloxane, alkyl-modified polysiloxane, and fluorine-modified polysiloxane; anionic surfactants such as fatty acid soap (such as sodium laurate or sodium palmitate), potassium lauryl sulfate, and alkyl sulfate triethanolamine ether; cationic surfactants such as stearyl trimethylammonium chloride, benzalkonium chloride, and lauryl amine oxide; amphoteric surfactants such as an imidazoline-based amphoteric surfactant (such as 2-cocoyl-2-imidazoliniumhydroxide-1-carboxyethyloxy disodium), a betaine-based surfactant (such as alkyl betaine, amido betaine, or sulfobetaine), and acyl methyl taurine; nonionic surfactants such as sorbitan fatty acid esters (such as sorbitan monostearate and sorbitan sesquioleate), glycerin fatty acid acids (such as glycerin monostearate), propylene glycol fatty acid esters (such as propylene glycol monostearate), a hydrogenated castor oil derivative, glycerin alkyl ether, POE sorbitan fatty acid esters (such as POE sorbitan monooleate and polyoxyethylene sorbitan monostearate), POE sorbitol fatty acid esters (such as POE-sorbitol monolaurate), POE glycerin fatty acid esters (such as POE-glycerin monoisostearate), POE fatty acid esters (such as polyethylene glycol monooleate and POE distearate), POE alkyl ethers (such as POE 2-octyl dodecyl ether), POE alkyl phenyl ethers (such as POE nonyl phenyl ether), pluronics, POE/POP alkyl ethers (such as POE/POP 2-decyl tetradecyl ether); tetronics; POE castor oil/hydrogenated castor oil derivative (such as POE castor oil and POE hydrogenated castor oil), sucrose fatty acid ester, and alkyl glucoside; polyhydric alcohols such as polyethylene glycol, glycerin, 1,3-butylene glycol, erythritol, sorbitol, xylitol, maltitol, propylene glycol, dipropylene glycol, diglycerin, isoprene glycol, 1,2-pentanediol, 2,4-hexanediol, 1,2-hexanediol, and 1,2-octanediol; a humectants such as sodium pyrrolidone carboxylate, lactic acid, and sodium lactate; powder which may be subjected to surface treatment such as mica, talc, kaolin, synthetic mica, calcium carbonate, magnesium carbonate, silicic anhydride (silica), aluminum oxide, and barium sulfate; inorganic pigments which may be subjected to surface treatment such as red oxide of iron, yellow oxide of iron, black oxide of iron, cobalt oxide, ultramarine, prussian blue, titanium oxide, and zinc oxide; pearling agents which may be subjected to surface treatment such as titanium mica, fish scale foil, and bismuth oxychloride; organic dyes which may be changed to lake such as Red No. 202, Red No. 228, Red No. 226, Yellow No. 4, Blue No. 404, Yellow No. 5, Red No. 505, Red No. 230, Red No. 223, Orange No. 201, Red No. 213, Yellow No. 204, Yellow No. 203, Blue No. 1, Green No. 201, Violet No. 201, and Red No. 204; organic powder such as polyethylene powder, methyl polymethacrylate, nylon powder, and an organopolysiloxane elastomer; a paraaminobenzoic acid-based UV absorbent; an anthranilic acid-based UV absorbent; a salicylic acid-based UV absorbent; a cinnamic acid-based UV absorbent; a benzophenone-based UV absorbent; a sugar-based UV absorbent; UV absorbents such as 2-(2'-hydroxy-5'-t-octylphenyl)benzotriazole and 4-methoxy-4'-t-butyldibenzoylmethane; lower alcohols such as ethanol and isopropanol; vitamin A and a derivative thereof; vitamin B such as a vitamin B₆ hydrochloride, vitamin B₆ tripalmitate, vitamin B₆ dioctanoate, vitamin B₂ or a derivative thereof, vitamin B₁₂, and vitamin B₁₅ or a derivative thereof; vitamin E such as α-tocopherol, β-tocopherol, γ-tocopherol, and vitamin E acetate; vitamins such as vitamin D, vitamin H, pantothenic acid, pantethin, and pyrroloquinoline quinone; and an antibacterial agent such as phenoxyethanol. Those components can be treated by a conventional method, to thereby produce the external preparation for skin of the second invention.

### Examples

Hereinafter, the present invention will be described in more detail with reference to examples. The present invention is obviously not limited to the examples.

### <Example 1>

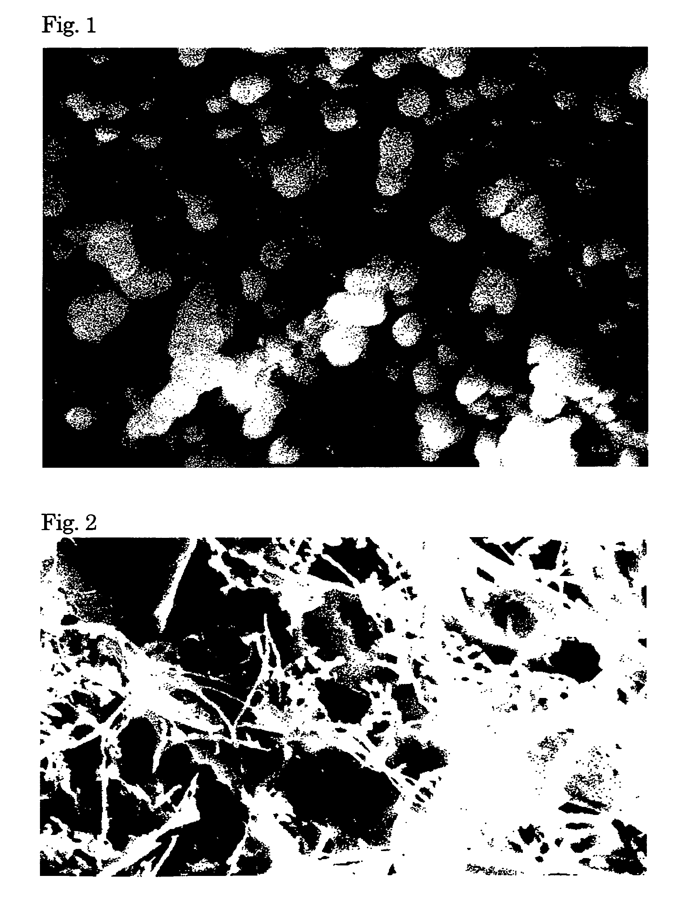

Zinc nitrate hexahydrate (18 g) and acrylic acid (1 g) were dissolved in a mixed solvent of methanol (134 g) and water (41 g), to thereby prepare Liquid A. 3 N caustic soda (56 g) was gradually introduced into Liquid A under stirring at room temperature, and the whole was heated immediately after a total amount was introduced. After the temperature had reached 50°C, azobisisobutyronitrile (0.02 g) was added. The resultant was continuously heated, maintained at a reflux temperature for 1 hour, and cooled. After cooling, an operation of decantation and filtration by using water was repeated three times, and an obtained precipitate was dried at 90°C for 4 hours, to thereby obtain a dried product (5.2 g). 95% of the dried product was wurzite zinc oxide. That is, the precipitate was a composite (Composite 1a) of fine particle zinc oxide and acrylic acid containing 95 mass% of spherical fine particle zinc oxide and 5 mass% of polyacrylic acid. Zinc oxide had a spherical shape and a particle size of 0.03 µm. Fig. 1 shows a microphotograph of this product. Fig. 1 reveals that spherical fine particle zinc oxide forms a composite with acrylic acid.

### <Example 2>

Zinc chloride (9 g) and polyacrylic acid (degree of polymerization of 5,000) (2 g) were dissolved in a mixed solvent of ethanol (116 g) and water (91 g), to thereby prepare Liquid A. 6 N caustic soda (31 g) was gradually introduced into Liquid A under stirring at room temperature, and the whole was heated 20 minutes after from a total amount was introduced, maintained at a reflux temperature for 1 hour, and cooled. After cooling, an operation of decantation and filtration by using water was repeated three times, and an obtained precipitate was dried at 90°C for 4 hours, to thereby obtain a dried product (5. 9 g). 85% % of the dried product was wurzite zinc oxide.

### <Example 3>

Iron chloride (10 g) and methyl methacrylic acid (2.3 g) were dissolved in a mixed solvent of ethoxyethanol (116 g) and water (140 g), to thereby prepare Liquid A. 6 N caustic soda (37 g) was gradually introduced into Liquid A under stirring at room temperature, and the whole was heated immediately after a total amount was introduced. After the temperature had reached 50°C, azobisisobutyronitrile (0.04 g) was added. The resultant was continuously heated, maintained at a reflux temperature for 1 hour, and cooled. After cooling, an operation of decantation and filtration by using water was repeated three times, and an obtained precipitate was dried at 90°C for 4 hours, to thereby obtain a dried product (5. 6 g). 75% of the dried product was Fe₂O₃ type iron oxide.

### <Example 4>

Titanium tetrachloride (12 g) and polyoxyethylene (9) acrylate (2.5 g) were dissolved in ethanol (165 g), to thereby prepare Liquid A. 6 N caustic soda (51 g) was gradually introduced into Liquid A under stirring at room temperature, and the whole was heated immediately after a total amount was introduced. After the temperature had reached 50°C, sodium peroxosulfate (0.04 g) was added, and the mixture was maintained at the same temperature for 16 hours. The resultant was filtered while the temperature was maintained at 60°C, and an operation of decantation and filtration by using separately prepared water at 60°C was repeated three times, and an obtained precipitate was dried at 90°C for 4 hours, to thereby obtain a dried product (5.1 g). 80% of the dried product was rutile-type titanium oxide.

### <Example 5>

Gold chloride (1 g) was dissolved in a mixed solvent of isopropanol (100 g) and water (300 g), to thereby prepare Liquid A. In addition, trisodium citrate (0.65 g) was dissolved in water (100 g), to thereby prepare Liquid B. Liquid A was heated to a reflux temperature, and Liquid B was dropped thereinto while Liquid A was stirred. The whole was maintained at the same temperature for 1 hour, and cooled. After cooling, an operation of decantation and filtration by using water was repeated three times, and an obtained precipitate was dried at 90°C for 4 hours, to thereby obtain a dried product (0.6 g). 90% of the dried product was colloidal gold.

### <Example 6>

The dried product (0.58 g) of Example 1 and water (300 mL) were introduced into a 500-mL beaker, and the whole was stirred at 200 rpm for 1 hour with a propeller stirrer (blade length of 4 cm), and dispersed, to thereby prepare Dispersion liquid A. 100 g of Dispersion liquid A was sampled, and water was added to a total amount of 1,000 g, to thereby obtain Aqueous paint 1A. A remainder of Dispersion liquid A was stirred at 6,000 rpm for 6 minutes (Condition 2) with a disperse stirrer (blade length of 3 cm) for redispersion. Then, 100 g of the resultant was sampled, and water was added to a total amount of 1, 000 g, to thereby obtain Aqueous paint 1B.

### <Example 7>

The dried product (0.176 g) of Example 2 was treated in the same manner as in Example 6, to thereby obtain Aqueous paints 2A and 2B.

### <Example 8>

The dried product (0.2 g) of Example 3 was treated in the same manner as in Example 6, to thereby obtain Aqueous paints 3A and 3B.

### <Example 9>

The dried product (0.188 g) of Example 4 was treated in the same manner as in Example 6, to thereby obtain Aqueous paints 4A and 4B.

### <Example 10>

The dried product (0.1 g) of Example 5 was treated in the same manner as in Example 6, to thereby obtain Aqueous paints 5A and 5B.

### <Test Example 1>

Comparative Examples 1 to 4 were produced through the following procedure, and an average particle size (µm) of each of Comparative Examples 1 to 4 was measured by using a laser diffraction scattering particle size distribution meter (wet). The average particle size (µm) of each of Aqueous paints 1A to 5B was measured in the same manner. Table 1 shows the results.

Table 1 reveals that each of Aqueous paints 1A to 5A and 1B to 5B employing the composition of the present invention produced by the production method of the present invention is easily dispersed as primary particles even with very weak stirring force regardless of stirring force during dispersion. This result indicates that inorganic particles are present independently from one another even in powder form. In contrast, commercially available inorganic particles of Comparative Examples 1 to 3 cannot be each dispersed as particles having a particle size of a catalogue value even under strong stirring force. That is, the commercially available inorganic particles in powder form form strong aggregates, and the aggregates cannot be broken easily.

### <Comparative Example 1>

0.15 g of commercially available fine particle zinc oxide powder (average particle size = 0.03 µm (catalogue value)) was sampled, and 0.01% sodium polyacrylate (degree of polymerization of 3,000) and water (300 mL) were introduced into a 500-mL beaker. The whole was stirred at 200 rpm for 1 hour with a propeller stirrer (blade length of 4 cm), and dispersed, to thereby prepare Dispersion liquid F. 100 g of Dispersion liquid F was sampled, and water was added to a total amount of 1,000 g, to thereby obtain Comparative Example 1A. A remainder of Dispersion liquid F was redispersed under Condition 2. 100 g of the resultant was sampled, and water was added to a total amount of 1,000 g, to thereby obtain Comparative Example 1B.

### <Comparative Example 2>

0.15 g of commercially available fine particle titanium oxide powder (average particle size = 0.023 µm (catalogue value) ) was sampled, and was treated in the same manner as in Comparative Example 1, to thereby obtain Comparative Examples 2A and 2B.

### <Comparative Example 3>

0.15 g of commercially available fine particle colcothar (red oxide of iron) powder (average particle size = 0.06 µm (catalogue value)) was sampled, and was treated in the same manner as in Comparative Example 1, to thereby obtain Comparative Examples 3A and 3B.

### <Comparative Example 4>

A commercially available gold colloid dispersion (average particle size = 0.01 µm (catalogue value)) was diluted with water by using a 10×10 mm cell such that a transmission of light of a wavelength of 700 nm was 80% through measurement with an integrating-sphere spectroscope, to thereby obtain Comparative Example 4B.

[Table 1]

**Table 1: Results of measurement of average particle size (µm)**

| | A | B |
|---|---|---|
| Example 6 | 0.033 | 0.031 |
| Example 7 | 0.025 | 0.020 |
| Example 8 | 0.030 | 0.028 |
| Example 9 | 0.220 | 0.190 |
| Example 10 | 0.018 | 0.016 |
| Comparative example 1 | 1.020 | 0.290 |
| Comparative example 2 | 0.960 | 0. 200 |
| Comparative example 3 | 1.200 | 0.470 |
| Comparative example 4 | - | 0.012 |

### <Example 11>

Zinc nitrate hexahydrate (18 g) and acrylic acid (1 g) were dissolved in a mixed solvent of methanol (134 g) and water (41 g), to thereby prepare Liquid A. 3 N caustic soda (56 g) was gradually introduced into Liquid A under stirring at room temperature, and the whole was heated immediately after a total amount was introduced. After the temperature had reached 50°C, azobisisobutyronitrile (0.02 g) was added. The resultant was continuously heated, maintained at a reflux temperature for 1 hour, and cooled. After cooling, an operation of decantation and filtration by using 50% water-containing ethanol was repeated three times, and an obtained precipitate was dried at 90°C for 4 hours, to thereby obtain a dried product (5.0 g). 95% of the dried product was wurzite zinc oxide. That is, the precipitate was a composite (Composite 1b) of fine particle zinc oxide and acrylic acid containing 95 mass% of fine particle zinc oxide in a whisker shape and 5 mass% of polyacrylic acid. Zinc oxide had a whisker shape, and the whisker had a minor axis of 0.03 µm and a major axis of 0.9 µm. Fig. 2 shows a microphotograph of this product.

### <Example 12>

A UV-protecting cosmetic (two-layer dispersed lotion form) as an external preparation for skin of the present invention was produced by using Composite 1b of Example 11 through a prescription shown in Table 2. That is, components of (i) were stirred and solubilized under heating at 80°C, and components of (ii) were dispersed thereinto, to thereby obtain UV-protecting cosmetic 1.

UV-protecting cosmetic 2 containing Composite 1a of Example 1 in place of Composite 1b of UV-protecting cosmetic 1 was produced in the same manner as that described above. Further, UV-protecting cosmetic 3 containing mixed powder including 95 mass% of commercially available fine particle zinc oxide powder (average particle size = 0.03 µm (catalogue value)) and 5 mass% of sodium polyacrylate in place of Composite 1b of UV-protecting cosmetic 1 was produced in the same manner as that described above.

[Table 2]

**Table 2**

| Components | Mass% |
|---|---|
| (i) | |
| 1,2-Pentanediol | 2 |
| 1,3-Butanediol | 5 |
| Ethanol | 5 |
| Phenoxyethanol | 0.4 |
| POE (20) behenyl ether | 0.5 |
| Sodium carboxymethyl cellulose | 0.1 |
| Water | 77 |
| (ii) | |
| Composite 1B | 10 |
| Total | 100 |

### <Test Example 2>

Sun protection factor (SPF) and protection grade of UV-A (PA) of each of UV-protecting cosmetics 1 to 3 were measured by using a back of a panelist in accordance with rules of Japan Cosmetic Industry Association. The results included: UV-protecting cosmetic 1 had SPF 20.3 and PA++; UV-protecting cosmetic 2 had SPF 17.5 and PA++; and UV-protecting cosmetic 3 had SPF 12.4 and PA+. SPF and PA of UV-protecting cosmetics 1 and 2 were higher than SPF and PA of UV-protecting cosmetic 3, and the results confirmed the effect of the composite of the present invention.

### <Test Example 3>

Whiteness of UV-protecting cosmetics 1 to 3 applied was evaluated. Three areas of 2 cm x 4 cm were prepared by using an inner forearm of a panelist, and 30 mg of each of the samples was applied to each area. After 5 minutes, brightness difference of those areas with respect to an untreated area was measured with a Konica Minolta colorimeter. The results included: UV-protecting cosmetic 1 had a brightness difference of 1.26; UV-protecting cosmetic 2 had a brightness difference of 2.03; and UV-protecting cosmetic 2 had a brightness difference of 3.69. The results revealed that the cosmetics of the present invention each had no white appearance.

### <Example 13>

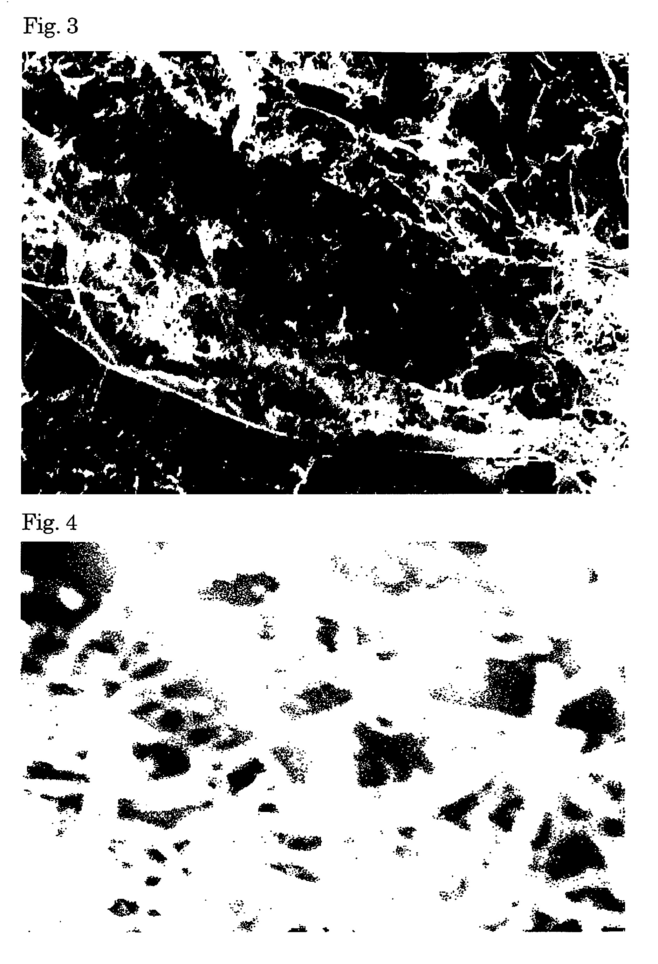

Zinc chloride (9 g) and polyacrylic acid (degree of polymerization of 5,000) (2 g) were dissolved in a mixed solvent of ethanol (116 g) and water (91 g), to thereby prepare Liquid A. 6 N caustic soda (31 g) was gradually introduced into Liquid A under stirring at room temperature, and the whole was heated 20 minutes after from a total amount was introduced, maintained at a reflux temperature for 1 hour, and cooled. After cooling, an operation of decantation and filtration by using 50% water-containing ethanol was repeated three times, and an obtained precipitate was dried at 90°C for 4 hours, to thereby obtain a dried product (5.3 g) as Composite 2b. 85% of the dried product was wurzite zinc oxide. The product was in a whisker shape having a minor axis of 0.02 µm and a major axis of 1.2 µm. Fig. 3 shows a microphotograph of this product.

### <Example 14>

UV-protecting cosmetic 4 as an external preparation for skin of the present invention was produced in the same manner as in Example 12 by using Composite 2b in accordance with following Table 3. UV-protecting cosmetic 4 had SPF 21.1 and PA++.

[Table 3]

**Table 3**

| Components | Mass% |
|---|---|
| (i) | |
| 1,2-Pentanediol | 2 |
| 1, 3-Butanediol | 5 |
| Ethanol | 5 |
| Phenoxyethanol | 0.4 |
| P0E (20) behenyl ether | 0.5 |
| Sodium carboxymethyl cellulose | 0.1 |
| Water | 77 |
| (ii) | |
| Composite 2B | 10 |
| Total | 100 |

### <Example 15>

Zinc chloride (9 g) and polyvinyl alcohol (degree of polymerization of 10,000) (2 g) were dissolved in a mixed solvent of ethanol (116 g) and water (91 g), to thereby prepare Liquid A. 6 N caustic soda (31 g) was gradually introduced into Liquid A under stirring at room temperature, and the whole was heated 20 minutes after from a total amount was introduced, maintained at a reflux temperature for 1 hour, and cooled. After cooling, an operation of decantation and filtration by using 50% water-containing ethanol was repeated three times, and an obtained precipitate was dried at 90°C for 4 hours, to thereby obtain a dried product (5.3 g) as Composite 3b. 85% of the dried product was wurzite zinc oxide. The product was in a whisker shape having a minor axis of 0.05 µm and a major axis of 0.9 µm. Fig. 4 shows a microphotograph of this product.

### Industrial Applicability

The present invention can be applied to paint, a cosmetic, and the like.

## Claims

1. An inorganic powder composite, comprising as components: inorganic fine particles; and one kind or two or more kinds of compounds selected from the group consisting of: a carboxylic acid; a carboxylic acid derivative; a carboxylic acid polymer; and a carboxylic acid derivative polymer each represented by the general formula (1), 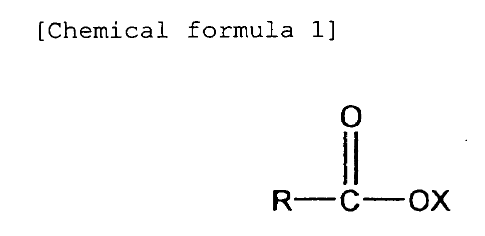 in the formula: R represents a hydrogen atom, or an alkyl group or alkenyl group which may have one or both of a carboxyl group and a hydroxyl group; and X represents hydrogen, an alkali metal, or polyoxyethylene having 23 or less carbon atoms.

2. The inorganic powder composite according to claim 1, wherein the carboxylic acid derivative represented by the general formula (1) comprises one kind or two or more kinds of compounds selected from the group consisting of: an alkali salt of a mono, di, or tricarboxylic acid having 10 or less carbon atoms; and a polyoxyethylene adduct of a mono, di, or tricarboxylic acid having 10 or less carbon atoms.

3. The inorganic powder composite according to claim 1 or 2, wherein the carboxylic acid derivative polymer represented by the general formula (1) comprises one kind or two or more kinds of compounds selected from the group consisting of an alkali salt of polyacrylic acid or polymethacrylic acid, and a polyoxyethylene adduct of polyacrylic acid or polymethacrylic acid.

4. The inorganic powder composite according to any one of claims 1 to 3, wherein
the inorganic fine particles comprises one kind of compound or a mixture of two or more kinds compounds selected from the group consisting of: a single metal; a metal oxide; and a metal hydroxide, and
the metal comprises one kind or two or more kinds of metals selected from the group consisting of: zinc; iron; aluminum; magnesium; titanium; barium; manganese; cerium; cobalt; calcium; cadmium; strontium; copper; chromium; zirconium; gold; and silver.

5. The inorganic powder composite according to any one of claims 1 to 4, wherein the inorganic fine particles have a particle size of 0.1 µm or less, and the inorganic fine particles are present independently from one another.

6. The inorganic powder composite according to any one of claims 1 to 5, wherein a percentage of the inorganic fine particles is 60 mass% or more.

7. An external preparation for skin, comprising the inorganic powder composite according to any one of claims 1 to 6.

8. An aqueous nail enamel, comprising the inorganic powder composite according to any one of claims 1 to 6.

9. An aqueous ink, comprising the inorganic powder composite according to any one of claims 1 to 6.

10. An aqueous coating material, comprising the inorganic powder composite according to any one of claims 1 to 6.

11. A method of producing the inorganic powder composite according to any one of claims 1 to 6, comprising:
dissolving a metal salt and one kind or two or more kinds of compounds selected from the group consisting of: a lower carboxylic acid represented by the general formula (1) and having X representing hydrogen; a polymer of the lower carboxylic acid; a carboxylic acid derivative represented by the general formula (1) and having X representing an atom excluding hydrogen; and a polymer of the carboxylic acid derivative in a water-miscible organic solvent or a mixed liquid of water and a water-miscible organic solvent; and
neutralizing the metal salt or reducing a metal of the metal salt; 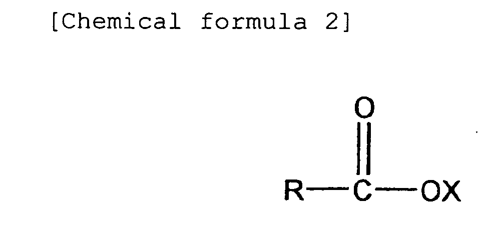
in the formula: R represents a hydrogen atom, or an alkyl group or alkenyl group which may have one or both of a carboxyl group and a hydroxyl group; and X represents hydrogen, an alkali metal, or polyoxyethylene having 23 or less carbon atoms.

12. The method according to claim 11, wherein
the compound represented by the general formula (1) comprises one or both of the lower carboxylic acid and a derivative thereof, and
the method further comprises polymerizing the lower carboxylic acid or the derivative thereof after neutralization of the metal salt or reduction of the metal.

13. The method according to claim 11 or 12, wherein the water-miscible organic solvent comprises one kind of compound or a mixture of two or more kinds of compounds selected from the group consisting of methanol, ethanol, and isopropanol.

14. The method according to any one of claims 11 to 13, wherein
the lower carboxylic acid represented by the general formula (1) and having X representing hydrogen comprises one kind or two or more kinds of compounds selected from the group consisting of mono, di, and tricarboxylic acids each having 10 or less carbon atoms, and
the carboxylic acid derivative represented by the general formula (1) comprises one kind or two or more kinds of polyoxyethylene adducts of lower carboxylic acids each having 10 or less carbon atoms.

15. The method according to any one of claims 11 to 14, wherein the polymer of the carboxylic acid derivative represented by the general formula (1) comprises one kind or two or more kinds of compounds selected from the group consisting of: an alkali salt of polyacrylic acid or polymethacrylic acid; and a polyoxyethylene adduct of polyacrylic acid or polymethacrylic acid.

16. The method according to any one of claims 11 to 15, wherein the metal salt comprises an inorganic acid salt of zinc, iron, aluminum, magnesium, titanium, barium, manganese, cerium, cobalt, calcium, cadmium, strontium, copper, chromium, zirconium, gold, or silver.

17. A method of producing a fine particle metal oxide or hydroxide/polymer composite, comprising the steps of:
reacting a metal salt and an alkali in the presence of a water-soluble polymer, or reacting a metal salt and an alkali in the presence of a water-soluble monomer and then polymerizing the water-soluble monomer, to form a composite of a metal oxide or hydroxide and the water-soluble polymer;
washing products excluding the composite with a water-containing alcohol; and
drying the resultant.

18. The method according to claim 17, wherein a fine particle metal oxide in the fine particle metal oxide/polymer composite is in a whisker shape.

19. The method according to claim 17 or 18, wherein a fine particle metal oxide in the fine particle metal oxide/polymer composite comprises zinc oxide.

20. The method according to any one of claims 17 to 19, wherein the water-soluble polymer comprises:
polyacrylic acid and/or a salt thereof; polymethacrylic acid and/or a salt thereof; or polyvinyl alcohol.

21. A fine particle metal oxide or hydroxide/polymer composite, comprising a composite of a water-soluble polymer and a fine particle metal oxide or hydroxide, wherein the metal oxide or hydroxide is present in a whisker shape.

22. The fine particle metal oxide or hydroxide/polymer composite according to claim 21, wherein the water-soluble polymer comprises: polyacrylic acid and/or a salt thereof; polymethacrylic acid and/or a salt thereof; or polyvinyl alcohol.

23. The fine particle metal oxide or hydroxide/polymer composite according to claim 21 or 22, wherein the fine particle metal oxide in the fine particle metal oxide/polymer composite comprises zinc oxide.

24. A fine particle metal oxide or hydroxide, which is a metal oxide or hydroxide in a whisker shape.

25. The fine particle metal oxide or hydroxide according to claim 24, wherein the fine particle metal oxide or hydroxide forms a composite with a water-soluble polymer and is present on the water-soluble polymer.

26. The fine particle metal oxide or hydroxide according to claim 24 or 25, wherein the fine particle metal oxide or hydroxide comprises zinc oxide.

27. An external preparation for skin, comprising the fine particle metal oxide or hydroxide/polymer composite according to any one of claims 21 to 23.

28. The external preparation for skin according to claim 27, wherein the external preparation for skin is in a water-containing form.

29. The external preparation for skin according to claim 27 or 28, wherein the external preparation for skin is a cosmetic.
